# EUROPEAN PATENT APPLICATION

(11) **EP 3 391 887 A1**
(43) Date of publication of application: **24.10.2018**
(21) Application number: 16875702.9
(22) Date of filing: 15.12.2016
(51) Int. Cl.: A61K 31/506, A61P 17/02

(54) **WOUND TREATMENT AGENT**

(30) Priority: 16.12.2015 JP 2015244951
(71) Applicant: Daiichi Sankyo Company, Limited, Tokyo 103-8426 (JP)
(72) Inventor: ISHII, Ken, Tokyo 103-8426 (JP)
(74) Representative: Wallace, Sheila Jane
(86) International application number: PCT/JP2016/087321
(87) International publication number: WO 2017/104725

(57) **Abstract**

The present invention provides a therapeutic agent for a wound comprising a compound represented by the formula (I) wherein R¹ represents a hydroxy C₁-C₆ alkyl group, a C₂-C₇ alkanoyl group, a C₂-C₇ alkanoyl C₁-C₆ alkyl group, a (C₁-C₆ alkoxy)carbonyl group, a (C₁-C₆ alkoxy)carbonyl C₁-C₆ alkyl group, a carboxy group, or a carboxy C₁-C₆ alkyl group, or a pharmacologically acceptable salt thereof as an active ingredient.

## Description

### Technical Field

The present invention relates to a therapeutic agent for a wound containing a 5-hydroxypyrimidine-4-carboxamide derivative as an active ingredient.

### Background Art

Wounds typified by skin ulcer, leg ulcer, burn ulcer, frostbite ulcer, traumatic ulcer, pressure ulcer, venous ulcer, arterial ulcer, immune ulcer, postherpetic ulcer, radiation induced ulcer, drug ulcer, diabetic ulcer, and postoperative ulcer, etc. are closed and healed through the proliferation of granulation tissues originating from angiogenesis at a wound site, and epidermal proliferation and elongation. However, if the patient's background disease or the environment of a wound site is not suitable for healing, delay in healing or exacerbation is often found. General procedures for chronic ulcers involve the physical or chemical removal of necrotic tissues, infection control using an antimicrobial agent, the irrigation of a wound site and the retention of a wet environment using a dressing. Many drugs have been developed so far with the aim of improving the environments of such wound sites, but these exert insufficient efficacy. Meanwhile, agents directly accelerating wound healing itself by angiogenesis induction are relatively few in number and are only two types: a bFGF formulation (Fiblast(R) spray) and a PDGF formulation (becaplermin gel). However, these agents are biologics and are very limited by their indications or areas of use. Hence, there is a demand for novel low molecular-weight drugs for treatment capable of directly accelerating wound healing. For example, gibberellin derivatives (Patent Reference 1), pseudopterosin derivatives (Patent Reference 2), sulfodehydroabietic acid (Patent Reference 3), and isoquinoline derivatives (Patent Reference 4) are currently known as low molecular-weight compounds having a wound healing accelerating effect.

Meanwhile, 5-hydroxypyrimidine-4-carboxamide derivatives are known to have excellent EPO production activity and are effective for the treatment of diseases caused by a decrease in EPO level (Patent References 5 and 6). However, it has not been known that 5-hydroxypyrimidine-4-carboxamide derivatives have a wound healing accelerating effect.

### Citation List

### Patent References

Patent Reference 1: International Publication No. WO 96/20703
Patent Reference 2: International Publication No. WO 96/40160
Patent Reference 3: Japanese Patent Laid-Open No. Hei10-338632
Patent Reference 4: Japanese Patent Laid-Open No. 2007-238458
Patent Reference 5: International Publication No. WO 2011/049126
Patent Reference 6: International Publication No. WO 2013/147214

### Summary of Invention

### Problems to be Resolved by the Invention

An object of the present invention is to provide a pharmaceutical containing a compound having an excellent wound healing accelerating effect.

### Means of Solving the Problem

The present inventors have conducted diligent studies to attain the object and consequently completed the present invention by finding that a 5-hydroxypyrimidine-4-carboxamide compound represented by the general formula (I) given below or a pharmacologically acceptable salt thereof (hereinafter, referred to as the compound of the present invention) has a strong angiogenic effect and granulation accelerating effect on a wound site and therefore has an excellent wound healing accelerating effect.

Specifically, the present invention provides:
(1) a therapeutic agent for a wound comprising a compound represented by the general formula (I): 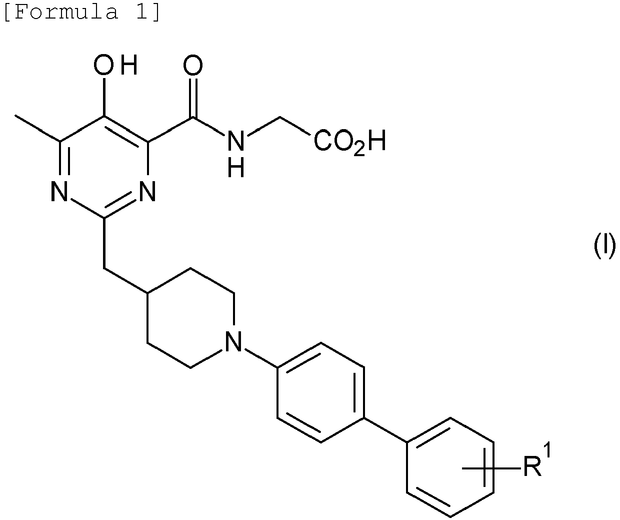 wherein
   R¹ represents a hydroxy C₁-C₆ alkyl group, a C₂-C₇ alkanoyl group, a C₂-C₇ alkanoyl C₁-C₆ alkyl group, a (C₁-C₆ alkoxy)carbonyl group, a (C₁-C₆ alkoxy)carbonyl C₁-C₆ alkyl group, a carboxy group, or a carboxy C₁-C₆ alkyl group,
   or a pharmacologically acceptable salt thereof as an active ingredient;
(2) a therapeutic agent for a wound according to (1), wherein R¹ is a carboxy group, a hydroxymethyl group, a 1-hydroxyethyl group, a 2-hydroxyethyl group, a 2-hydroxypropyl group, a 3-hydroxypropyl group, a 2-hydroxybutyl group, an acetyl group, a methoxycarbonyl group, an ethoxycarbonyl group, a 2-oxopropyl group, a 2-oxobutyl group, a 3-oxobutyl group, a 2-oxopentyl group, a methoxycarbonylmethyl group, or a carboxymethyl group;
(3) a therapeutic agent for a wound according to (1), wherein R¹ is a carboxy group, a hydroxymethyl group, a 1-hydroxyethyl group, a 2-hydroxyethyl group, a 2-hydroxypropyl group, a 3-hydroxypropyl group, a 2-hydroxybutyl group, a 2-oxopropyl group, a 2-oxobutyl group, a 3-oxobutyl group, or a 2-oxopentyl group;
(4) a therapeutic agent for a wound according to (1), wherein the compound represented by the general formula (I) is one compound selected from the group consisting of
   ({[5-hydroxy-2-({1-[4'-(hydroxymethyl)biphenyl-4-yl]piperidin-4-yl}methyl)-6-methylpyrimidin-4-yl]carbonyl}amino)acetic acid,
   ({[5-hydroxy-2-({1-[4'-(2-hydroxypropyl)biphenyl-4-yl]piperidin-4-yl}methyl)-6-methylpyrimidin-4-yl]carbonyl}amino)acetic acid,
   [({5-hydroxy-2-[(1-{4'-[(2S)-2-hydroxypropyl]biphenyl-4-yl}piperidin-4-yl)methyl]-6-methylpyrimidin-4-yl}carbonyl)amino]acetic acid,
   [({5-hydroxy-2-[(1-{4'-[(2R)-2-hydroxypropyl]biphenyl-4-yl}piperidin-4-yl)methyl]-6-methylpyrimidin-4-yl}carbonyl)amino]acetic acid,
   ({[5-hydroxy-6-methyl-2-({1-[4'-(2-oxopropyl)biphenyl-4-yl]piperidin-4-yl}methyl)pyrimidin-4-yl]carbonyl}amino)acetic acid, and
   4'-[4-({4-[(carboxymethyl)carbamoyl]-5-hydroxy-6-methylpyrimidin-2-yl}methyl)piperidin-1-yl]biphenyl-4-carboxylic acid;
(5) a therapeutic agent for a wound according to any one of (1) to (4), wherein the wound is selected from the group consisting of a skin ulcer, a leg ulcer, a burn ulcer, a frostbite ulcer, a traumatic ulcer, a pressure ulcer , a venous ulcer, an arterial ulcer, an immune ulcer, a postherpetic ulcer, a radiation induced ulcer, a drug ulcer, a diabetic ulcer, and a postoperative ulcer;
(6) a therapeutic agent for a wound according to any one of (1) to (4), wherein the wound is selected from the group consisting of a burn ulcer, a pressure ulcer , and a diabetic foot ulcer;
(7) a method for treating a wound, comprising administering a therapeutic agent for a wound according to any one of (1) to (6);
(8) a method according to (7), wherein the wound is selected from the group consisting of a skin ulcer, a leg ulcer, a burn ulcer, a frostbite ulcer, a traumatic ulcer, a pressure ulcer, a venous ulcer, an arterial ulcer, an immune ulcer, a postherpetic ulcer, a radiation induced ulcer, a drug ulcer, a diabetic ulcer, and a postoperative ulcer;
(9) a method according to (7), wherein the wound is selected from the group consisting of a burn ulcer, a pressure ulcer, and a diabetic foot ulcer;
(10) a therapeutic agent for a wound according to any one of (1) to (6), wherein the therapeutic agent shortens a period required for the healing of the wound;
(11) a therapeutic agent for a wound according to any one of (1) to (6) for use in a method for treating a wound; and
(12) a method for accelerating the healing of a wound, comprising administering a therapeutic agent for a wound according to any one of (1) to (6).

### Advantageous Effects of Invention

The compound of the present invention represented by the general formula (I) has a strong angiogenic effect and granulation accelerating effect on a wound site and is therefore useful as a pharmaceutical active ingredient, particularly, as an active ingredient in a therapeutic agent for a wound. Thus, the therapeutic agent for a wound of the present invention can be used in the treatment of a skin ulcer, a leg ulcer, a burn ulcer, a frostbite ulcer, a traumatic ulcer, a pressure ulcer , a venous ulcer, an arterial ulcer, an immune ulcer, a postherpetic ulcer, a radiation induced ulcer, a drug ulcer, a diabetic ulcer, or a postoperative ulcer, particularly, in the treatment of a burn ulcer, a pressure ulcer or a diabetic foot ulcer.

### Brief Description of the Drawings

[Figure 1] Figure 1 is a diagram showing time dependent change in the percent wound areas of a compound A administration group and a negative control group in wound model mice.
[Figure 2] Figure 2 is a diagram showing the area under the curve (AUC, % × day) of the percent wound areas of a compound A administration group and a negative control group in wound model mice.
[Figure 3] Figure 3 is a diagram showing time dependent change in the percent wound areas of a compound B administration group, a bFGF administration group and a negative control group in wound model mice.
[Figure 4A] Figure 4A is a diagram showing the area under the curve (AUC, % × day) of the percent wound areas of a compound B administration group, a bFGF administration group and a negative control group in wound model mice.
[Figure 4B] Figure 4B is a diagram showing the number of days required for 50% reduction in the percent wound areas of a compound B administration group, a bFGF administration group and a negative control group in wound model mice.
[Figure 4C] Figure 4C is a diagram showing 75% healing achievement at day 14 in a compound B administration group, a bFGF administration group and a negative control group in wound model mice.
[Figure 5] Figure 5 is a diagram showing time dependent change in the percent wound areas of a compound A administration group and a negative control group in chronic wound model rats.
[Figure 6A] Figure 6A is a diagram showing the area under the curve (AUC, % × day) of the percent wound areas of a compound A administration group and a negative control group in chronic wound model rats.
[Figure 6B] Figure 6B is a diagram showing the number of days required for healing with a compound A administration group and a negative control group in chronic wound model rats.
[Figure 6C] Figure 6C is a diagram showing time dependent change in the complete healing rates of a compound A administration group and a negative control group in chronic wound model rats.

### Description of the Embodiments

In the present invention, the "therapeutic agent for a wound" refers to an agent having a wound healing accelerating effect.

In the present invention, the "acceleration of wound healing" refers to the shortening of a period required for the healing of the wound.

The therapeutic agent for a wound of the present invention contains a compound represented by the general formula (I) described above or a pharmacologically acceptable salt thereof as an active ingredient.

Hereinafter, substituents related to the compound represented by the general formula (I) contained in the therapeutic agent for a wound of the present invention will be described.

The term "C₁-C₆ alkyl group" refers to a linear or branched alkyl group having 1 to 6 carbon atoms. Examples thereof can include a methyl group, an ethyl group, a propyl group, an isopropyl group, a butyl group, a sec-butyl group, a tert-butyl group, a pentyl group, an isopentyl group, a 2-methylbutyl group, a neopentyl group, a 1-ethylpropyl group, a hexyl group, a 4-methylpentyl group, a 3-methylpentyl group, a 2-methylpentyl group, a 1-methylpentyl group, a 3,3-dimethylbutyl group, a 2,2-dimethylbutyl group, a 1,1-dimethylbutyl group, a 1,2-dimethylbutyl group, a 1,3-dimethylbutyl group, a 2,3-dimethylbutyl group, and a 2-ethylbutyl group.

The term "hydroxy C₁-C₆ alkyl group" in the definition of R¹ refers to a group in which one or more hydrogen atoms (preferably, 1 or 2 hydrogen atom(s)) of a "C₁-C₆ alkyl group" are replaced with a hydroxy group. Examples thereof can include a hydroxymethyl group, a 1-hydroxyethyl group, a 2-hydroxyethyl group, a 1-hydroxypropyl group, a 2-hydroxypropyl group, a 3-hydroxypropyl group, a 2-hydroxy-1,1-dimethylethyl group, a 2-hydroxybutyl group, and a 2-hydroxypentyl group. The hydroxy C₁-C₆ alkyl group is preferably a hydroxymethyl group, a 1-hydroxyethyl group, a 2-hydroxyethyl group, a 2-hydroxypropyl group, a 3-hydroxypropyl group, or a 2-hydroxybutyl group, more preferably a hydroxymethyl group or a 2-hydroxypropyl group.

The term "C₂-C₇ alkanoyl group" in the definition of R¹ refers to a group in which a "C₁-C₆ alkyl group" is bonded to a carbonyl group. Examples thereof can include an acetyl group, a propionyl group, a butyryl group, an isobutyryl group, a pentanoyl group, a pivaloyl group, a valeryl group, an isovaleryl group, a hexanoyl group, and a heptanoyl group. The C₂-C₇ alkanoyl group is preferably an acetyl group.

The term "C₂-C₇ alkanoyl C₁-C₆ alkyl group" in the definition of R¹ refers to a group in which one hydrogen atom of a "C₁-C₆ alkyl group" is replaced with a "C₂-C₇ alkanoyl group". Examples thereof can include a 2-oxopropyl group, a 2-oxobutyl group, a 3-oxobutyl group, a 2-oxopentyl group, a 3-oxopentyl group, and a 4-oxopentyl group. The C₂-C₇ alkanoyl C₁-C₆ alkyl group is preferably a 2-oxopropyl group, a 2-oxobutyl group, a 3-oxobutyl group, or a 2-oxopentyl group, more preferably a 2-oxopropyl group.

The term "C₁-C₆ alkoxy group" in the definition of R¹ refers to a group in which a "C₁-C₆ alkyl group" is bonded to an oxygen atom. Examples thereof can include a methoxy group, an ethoxy group, a n-propoxy group, a n-butoxy group, a s-butoxy group, a tert-butoxy group, and a n-pentoxy group.

The term "(C₁-C₆ alkoxy)carbonyl group" in the definition of R¹ refers to a group in which a "C₁-C₆ alkoxy group" is bonded to a carbonyl group. Examples thereof can include a methoxycarbonyl group, an ethoxycarbonyl group, a n-propoxycarbonyl group, and a n-butoxycarbonyl group. The (C₁-C₆ alkoxy)carbonyl group is preferably a methoxycarbonyl group or an ethoxycarbonyl group.

The term "(C₁-C₆ alkoxy) carbonyl C₁-C₆ alkyl group" in the definition of R¹ refers to a group in which a " (C₁-C₆ alkoxy) carbonyl group" is bonded to a "C₁-C₆ alkyl group". Examples thereof can include a methoxycarbonylmethyl group, a methoxycarbonylethyl group, an ethoxycarbonylmethyl group, an ethoxycarbonylethyl group, a n-propoxycarbonylmethyl group, a n-propoxycarbonylethyl group, a n-butoxycarbonylmethyl group, and a n-butoxycarbonylethyl group. The (C₁-C₆ alkoxy)carbonyl C₁-C₆ alkyl group is preferably a methoxycarbonylmethyl group.

The term "carboxy C₁-C₆ alkyl group" in the definition of R¹ refers to a group in which a carboxy group is bonded to a "C₁-C₆ alkyl group". Examples thereof can include a carboxymethyl group, a 1-carboxyethyl group, a 2-carboxyethyl group, a 1-carboxypropyl group, a 2-carboxypropyl group, a 3-carboxypropyl group, a 2-carboxy-1,1-dimethylethyl group, a 2-carboxybutyl group, and a 2-carboxypentyl group. The carboxy C₁-C₆ alkyl group is preferably a carboxymethyl group.

In the present invention, R¹ preferably refers to a carboxy group, a hydroxymethyl group, a 1-hydroxyethyl group, a 2-hydroxyethyl group, a 2-hydroxypropyl group, a 3-hydroxypropyl group, a 2-hydroxybutyl group, an acetyl group, a methoxycarbonyl group, an ethoxycarbonyl group, a 2-oxopropyl group, a 2-oxobutyl group, a 3-oxobutyl group, a 2-oxopentyl group, a methoxycarbonylmethyl group, or a carboxymethyl group, more preferably a carboxy group, a hydroxymethyl group, a 1-hydroxyethyl group, a 2-hydroxyethyl group, a 2-hydroxypropyl group, a 3-hydroxypropyl group, a 2-hydroxybutyl group, a methoxycarbonyl group, an ethoxycarbonyl group, a 2-oxopropyl group, a 2-oxobutyl group, a 3-oxobutyl group, or a 2-oxopentyl group, even more preferably a carboxy group, a hydroxymethyl group, a 2-hydroxypropyl group, or a 2-oxopropyl group.

The compound described in formula (I) of the present invention is preferably one compound selected from the following compounds or pharmacologically acceptable salts thereof:
({[5-hydroxy-2-({1-[4'-(hydroxymethyl)biphenyl-4-yl]piperidin-4-yl}methyl)-6-methylpyrimidin-4-yl]carbonyl}amino)acetic acid,
({[5-hydroxy-2-({1-[4'-(2-hydroxypropyl)biphenyl-4-yl]piperidin-4-yl}methyl)-6-methylpyrimidin-4-yl]carbonyl}amino)acetic acid,
[({5-hydroxy-2-[(1-{4'-[(2S)-2-hydroxypropyl]biphenyl-4-yl}piperidin-4-yl)methyl]-6-methylpyrimidin-4-yl}carbonyl)amino]acetic acid,
[({5-hydroxy-2-[(1-{4'-[(2R)-2-hydroxypropyl]biphenyl-4-yl}piperidin-4-yl)methyl]-6-methylpyrimidin-4-yl}carbonyl)amino]acetic acid,
({[5-hydroxy-6-methyl-2-({1-[4'-(2-oxopropyl)biphenyl-4-yl]piperidin-4-yl}methyl)pyrimidin-4-yl]carbonyl}amino)acetic acid, and
4'-[4-({4-[(carboxymethyl)carbamoyl]-5-hydroxy-6-methylpyrimidin-2-yl}methyl)piperidin-1-yl]biphenyl-4-carboxylic acid.

When the compound represented by the general formula (I) of the present invention has an asymmetric carbon atom, optical isomers may be present. The present invention encompasses forms (e.g., enantiomers or diastereomers) resolved from these isomers, or mixtures thereof (e.g., racemic or diastereomeric mixtures).

The compound represented by the general formula (I) of the present invention, when having a basic group such as an amino group, can form a pharmacologically acceptable acid addition salt, if desired. Examples of such an acid addition salt can include: hydrohalides such as hydrofluoride, hydrochloride, hydrobromide, and hydroiodide; inorganic acid salts such as nitrate, perchlorate, sulfate, and phosphate; lower alkanesulfonates such as methanesulfonate, trifluoromethanesulfonate, and ethanesulfonate; arylsulfonates such as benzenesulfonate and p-toluenesulfonate; organic acid salts such as acetic acid, malic acid, fumarate, succinate, citrate, tartrate, oxalate, and maleate; and amino acid salts such as ornithine acid salt, glutamate, and aspartate. Hydrohalides and organic acid salts are preferred.

The compound represented by the general formula (I) of the present invention, when having an acidic group such as a carboxy group, is generally capable of forming a pharmacologically acceptable base addition salt. Examples of such a base addition salt can include: alkali metal salts such as sodium salt, potassium salt, and lithium salt; alkaline earth metal salts such as calcium salt and magnesium salt; inorganic salts such as ammonium salt; and organic amine salts such as dibenzylamine salt, morpholine salt, phenylglycine alkyl ester salt, ethylenediamine salt, N-methylglucamine salt, diethylamine salt, triethylamine salt, cyclohexylamine salt, dicyclohexylamine salt, N,N'-dibenzylethylenediamine salt, diethanolamine salt, N-benzyl-N-(2-phenylethoxy)amine salt, piperazine salt, tetramethylammonium salt, and tris(hydroxymethyl)aminomethane salt.

The compound represented by the general formula (I) of the present invention may exist as a non-solvate or a solvate. The solvate is not particularly limited as long as the solvate is pharmacologically acceptable. Specifically, a hydrate, an ethanol solvate, or the like is preferred.

The compound represented by the general formula (I) of the present invention can be produced by, for example, a method described in WO2011/049126 or WO2013/147214.

The therapeutic agent for a wound of the present invention is not particularly limited by its administration method and can be administered orally or parenterally by a method suitable for various dosage forms, the age, sex, and other conditions of a patient, the severity of the disease, etc. Examples of the dosage forms include tablets, pills, capsules, granules, powders, suspensions, emulsions, and solutions for oral administration and include local administration formulations, injections, percutaneous formulations, suppositories, transnasal formulations, and inhalants for parenteral administration. Local administration formulations are preferred. Examples of local administration formulations include sprays, aerogels, creams, ointments, lotions, liniments, and gels. Sprays, creams, ointments, and gels are preferred.

The dose and the number of doses of the therapeutic agent for a wound of the present invention are appropriately selected according to a dose regimen, the age, sex, and other conditions of a patient, and the severity of the disease. For example, the dose is usually 0.01 mg/kg to 100 mg/kg per dose in an adult, and the number of doses is usually once to six times a day. The content of the active ingredient in the formulation is usually 0.0001 to 1% by weight, preferably 0.001 to 0.1% by weight, more preferably 0.01 to 0.03% by weight. The formulation can be supplemented, if necessary, with additives such as an absorption enhancer, a pH adjuster, a preservative, a flavoring agent, a dispersant, a wetting agent, a stabilizer, an antiseptic, a suspending agent, and a surfactant.

The therapeutic agent for a wound of the present invention can be administered together with an antimicrobial drug, a disinfectant, or a necrotic tissue removing drug.

Examples of the antimicrobial drug include povidone iodine sugar (U-PASTA(trademark)), sulfadiazine silver (GEBEN(trademark)), and cadexomer iodine (CADEX(trademark)).

Examples of the disinfectant include povidone iodine, chlorohexidine gluconate, and benzalkonium chloride.

Examples of the necrotic tissue removing drug include bromelain, cadexomer iodine, dextranomer, sulfadiazine silver, hydrogel, and fradiomycin sulfate-trypsin crystallized mixture.

The therapeutic agent for a wound of the present invention can be used together with a dressing. Examples of the dressing can include gauze, polyurethane films, hydrocolloids, polyurethane foam, alginate dressings, hydrogel, hydrofiber, and hydropolymer.

### Examples

Hereinafter, the present invention will be described in more detail with reference to the Examples, Test Examples and Formulation Example. However, the scope of the present invention is not limited by these examples.

### (Example 1)

### {[5-Hydroxy-2-({1-[4'-(hydroxymethyl)biphenyl-4-yl]piperidin-4-yl}methyl)-6-methylpyrimidin-4-yl]carbonyl}amino)acetic acid (compound A)

Compound A was produced according to the method of Example 1 of WO2011/049126.

### (Example 2)

### [({5-Hydroxy-2-[(1-{4'-[(2S)-2-hydroxypropyl]biphenyl-4-yl}piperidin-4-yl)methyl]-6-methylpyrimidin-4-yl}carbonyl)amino]acetic acid (compound B)

Compound B was produced according to the method of Example 45 of WO2011/049126.

### (Test Example 1) Efficacy of compound A in a wound healing experiment

### [Method]

A full thickness skin wound was prepared on the backs of 9-week-old male db/db mice. The back of each mouse was shaved with an electric shaver under isoflurane anesthesia, and a full thickness skin wound of 8 mm in diameter was created on the back with a disposable biopsy punch. Compound A was administered dropwise (0.04 mL per wound site) at a concentration of 0.5 mM to the wound site once a day over 4 days from the wound creation date and protected with a dressing (Tegaderm Transparent Dressing). The negative control used was a test substance solubilizing solvent (10% Lutrol F127, 2% propylene glycol, 20 mM Tris buffer (pH 8.5)). 1, 4, 7, 9, 11, 15, and 18 days after the wound creation date, the wound site was photographed with a digital camera, and the wound area was calculated using image analysis software. When the wound area of the wound creation date was defined as 100%, the area at each day of photographing was calculated in terms of the percentage (%) of the area of the wound creation date. The test was conducted with 8 animals per group.

### [Results]

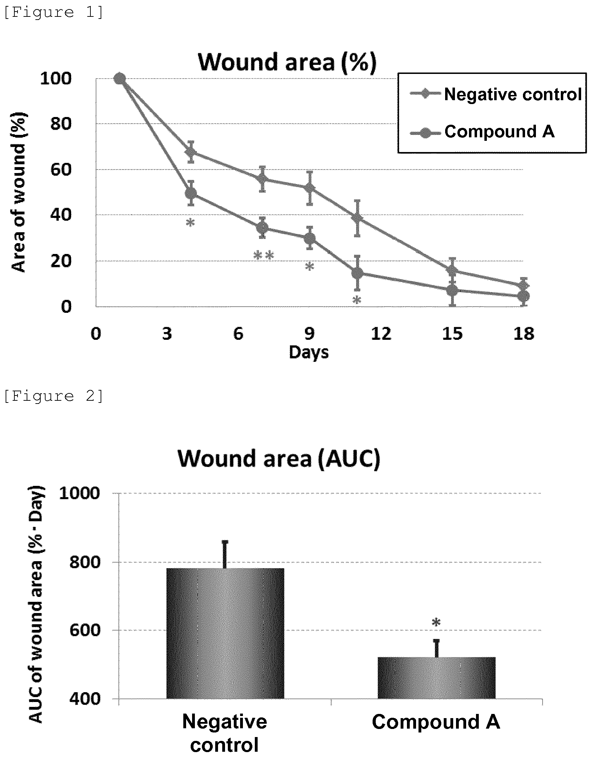

Figure 1 shows time-dependent change in the percent wound area. Compound A reduced the percent wound area at each point of evaluation, as compared with the negative control. The administration of compound A exhibited a significant wound healing accelerating effect.

Figure 2 shows the area under the curve (AUC, % × day) of the percent wound area calculated from the time-dependent change in the percent wound area. AUC of the compound A administration group exhibited a low value as compared with AUC of the negative control group. Thus, compound A significantly accelerated wound healing.

### (Test Example 2) Efficacy of compound B in a wound healing experiment

### [Method]

A full thickness skin wound was prepared on the backs of 10-week-old male db/db mice. The back of each mouse was shaved with an electric shaver under isoflurane anesthesia, and a full thickness skin wound of 8 mm in diameter was created on the back with a disposable biopsy punch. When the wound creation date was defined as day 1, compound B was administered dropwise (0.04 mL per wound site) at a concentration of 0.15 mM to the wound site once a day at days 1, 2, 4, 7, 10, 14, 17, and 22 and protected with a dressing (Tegaderm Transparent Dressing). A positive control bFGF (trafermin (genetic recombination) formulation, Fiblast(trademark) spray) approved as a therapeutic agent for pressure ulcer and skin ulcer was sprayed five times (approximately 30 µg was administered per 6 cm in diameter) to the wound site from approximately 5 cm apart according to the dose regimen. The negative control used was a test substance solubilizing solvent (10% Lutrol F127, 2% propylene glycol, 20 mM Tris buffer (pH 8.5)). At the test substance administration dates (days 1, 2, 4, 7, 10, 14, 17, and 22) and day 24, the wound site was photographed with a digital camera, and the wound area was calculated using image analysis software. When the wound area of day 1 was defined as 100%, the area at each day of photographing was calculated in terms of the percentage (%) of the area of the wound creation date. The test was conducted with 6 animals per group.

### [Results]

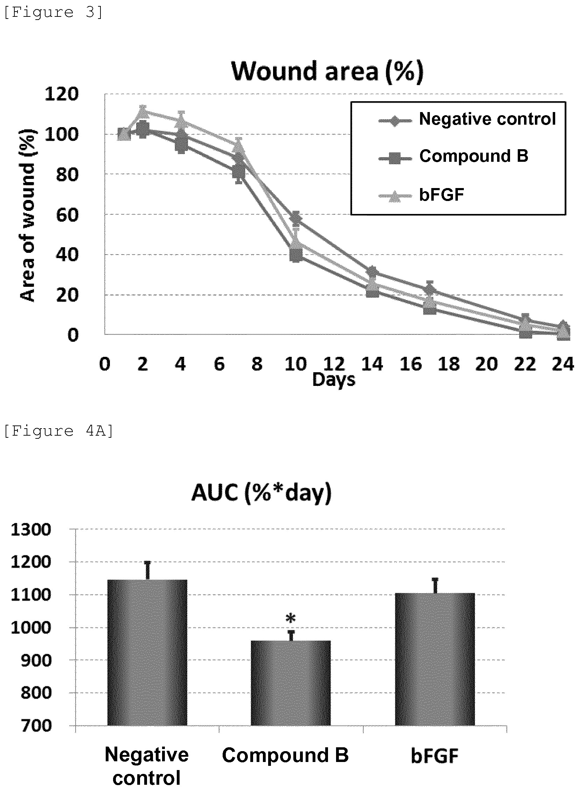

Figure 3 shows time dependent change in the percent wound area. Compound B reduced the percent wound area at each point of evaluation, as compared with the negative control. The administration of compound B exhibited a significant wound healing accelerating effect.

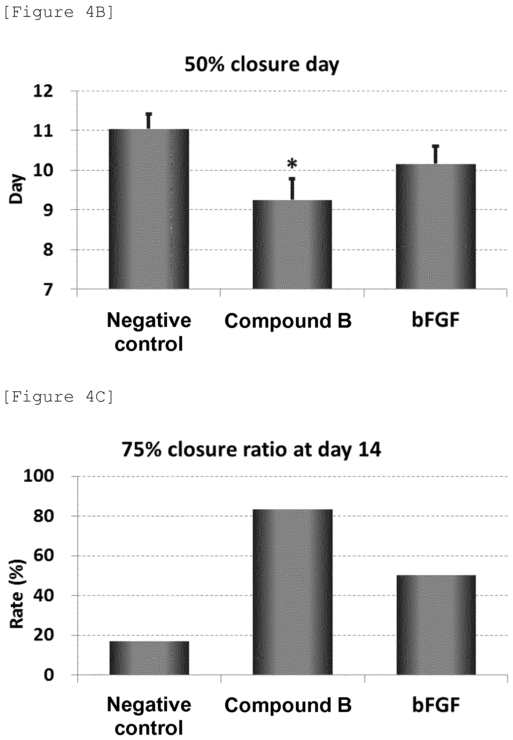

Figure 4A shows the area under the curve (AUC, % × day) of the percent wound area calculated from the time dependent change in the percent wound area. AUC of the compound B administration group exhibited a low value as compared with AUC of the negative control group. Thus, compound B significantly accelerated wound healing. AUC of the compound B administration group also exhibited a low value as compared with the positive control bFGF administration group. Figure 4B shows the number of days required for 50% reduction in the percent wound area. The number of days required for 50% reduction in the percent wound area was 11.0 days for the negative control group, 9.2 days for the compound B group, and 10.2 days for the bFGF administration group and was thus significantly shortened by the administration of compound B. Figure 4C shows the proportion of individuals that achieved 75% or more reduction in the percent wound area at day 14 (75% healing rate). The 75% healing rate was 16.7% for the negative control group, 83.3% for the compound B group, and 50.0% for the bFGF administration group. These results indicate that the efficacy of compound B was superior in wound healing to bFGF.

### (Test Example 3) Efficacy of compound A in a chronic wound healing experiment

### [Method]

Pressure ulcer was created by pressure load on the right third trochanters of 8-week-old male SD rats. The skin on the right third trochanter of each rat was shaved with an electric shaver. A pressure of 902.3 to 911.2 g/cm² was loaded on the right third trochanter for 24 hours under anesthesia with pentobarbital and allobarbital. 2 days after cancellation of the pressure load, necrotic tissues were surgically removed under isoflurane anesthesia. When the necrotic tissue removal date was defined as day 1, compound A was administered dropwise (0.02 to 0.06 mL per wound site) at a concentration of 0.5 mM to the wound site once a day and protected with gauze and a waterproof film. The negative control used was a test substance solubilizing solvent (10% Lutrol F127, 2% propylene glycol, 20 mM Tris buffer (pH 8.5)). The major axis and minor axis of the wound surface were measured every day with a vernier caliper, and the wound area (major axis × minor axis) was calculated. When the wound area of day 1 was defined as 100%, the calculated area at each day was calculated in terms of the percentage (%) of the area of the wound creation date. The test was conducted with 10 animals per group.

### [Results]

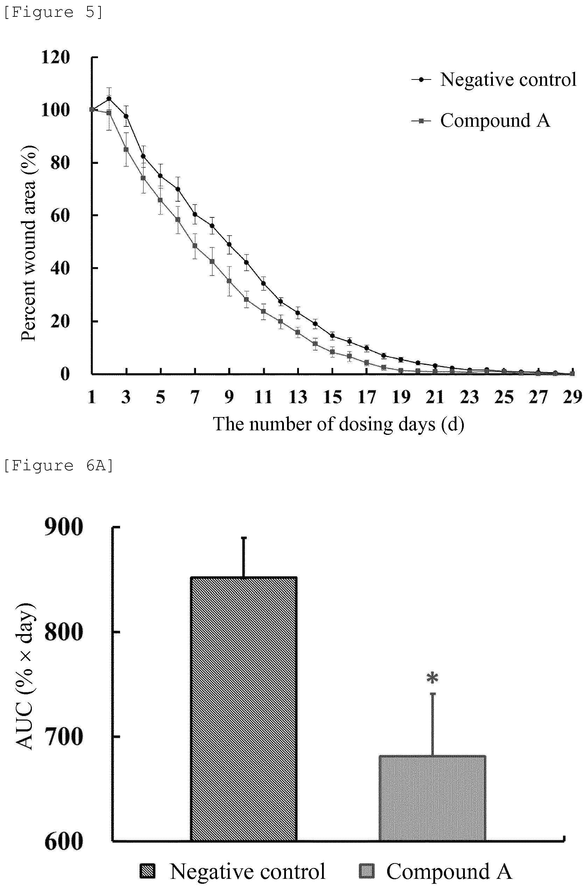

Figure 5 shows time dependent change in the percent wound area. Compound A reduced the percent wound area at each point of evaluation, as compared with the negative control. The administration of compound A exhibited a significant wound healing accelerating effect.

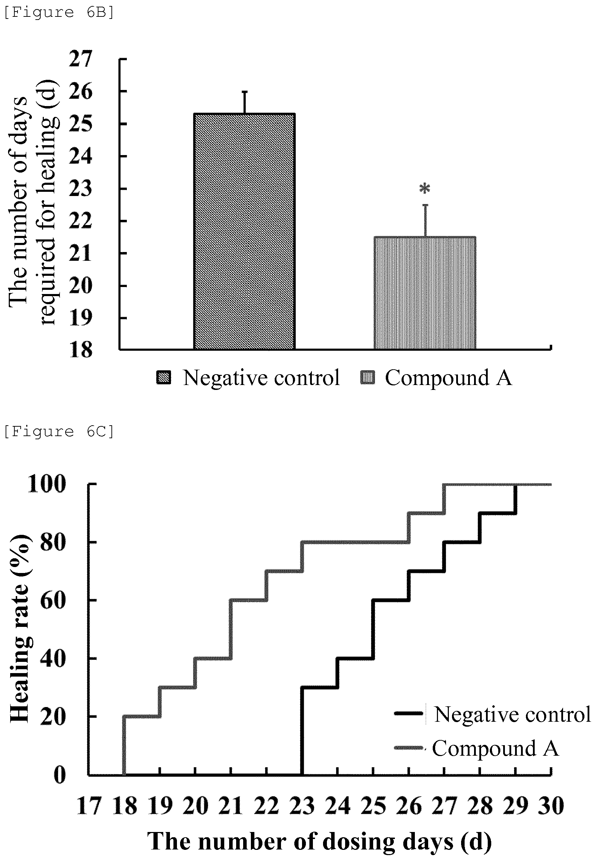

Figure 6A shows the area under the curve (AUC, % × day) of the percent wound area calculated from the time dependent change in the percent wound area. AUC of the compound A administration group exhibited a low value as compared with AUC of the negative control group. Thus, compound A significantly accelerated wound healing. Figure 6B shows the number of days required for healing. The number of days required for healing was 25.3 days for the negative control group and 21.5 days for the compound A group and was thus significantly shortened by the administration of compound A. Figure 6C shows time dependent change in the healing rate. The compound A group exhibited significant elevation in the healing rate as compared with the negative control group.

### (Formulation Example)

### Formulation Example 1 (solution)

A compound of Example (10 mg) is dissolved in ethanol (1 ml) and subsequently diluted with distilled water (99 ml) to prepare a solution.

### Industrial Applicability

The compound of the present invention represented by the general formula (I) has a strong angiogenic effect and granulation accelerating effect on a wound site and is therefore useful as a pharmaceutical active ingredient, particularly, as an active ingredient in a therapeutic agent for a wound. Thus, the therapeutic agent for a wound of the present invention can be used in the treatment of a skin ulcer, a leg ulcer, a burn ulcer, a frostbite ulcer, a traumatic ulcer, a pressure ulcer, a venous ulcer, an arterial ulcer, an immune ulcer, a postherpetic ulcer, a radiation induced ulcer, a drug ulcer, a diabetic ulcer, or a postoperative ulcer, particularly, the treatment of a burn ulcer, a pressure ulcer or a diabetic foot ulcer.

## Claims

1. A therapeutic agent for a wound comprising a compound represented by the general formula (I): 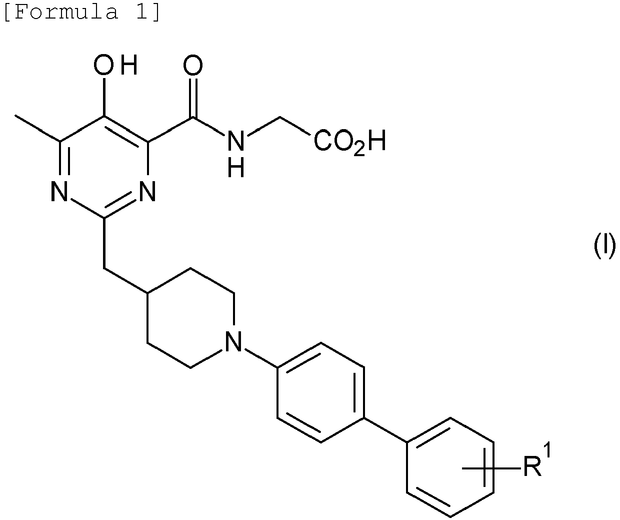 wherein
R¹ represents a hydroxy C₁-C₆ alkyl group, a C₂-C₇ alkanoyl group, a C₂-C₇ alkanoyl C₁-C₆ alkyl group, a (C₁-C₆ alkoxy)carbonyl group, a (C₁-C₆ alkoxy)carbonyl C₁-C₆ alkyl group, a carboxy group, or a carboxy C₁-C₆ alkyl group,
or a pharmacologically acceptable salt thereof as an active ingredient.

2. A therapeutic agent for a wound according to claim 1, wherein R¹ is a carboxy group, a hydroxymethyl group, a 1-hydroxyethyl group, a 2-hydroxyethyl group, a 2-hydroxypropyl group, a 3-hydroxypropyl group, a 2-hydroxybutyl group, an acetyl group, a methoxycarbonyl group, an ethoxycarbonyl group, a 2-oxopropyl group, a 2-oxobutyl group, a 3-oxobutyl group, a 2-oxopentyl group, a methoxycarbonylmethyl group, or a carboxymethyl group.

3. A therapeutic agent for a wound according to claim 1, wherein R¹ is a carboxy group, a hydroxymethyl group, a 1-hydroxyethyl group, a 2-hydroxyethyl group, a 2-hydroxypropyl group, a 3-hydroxypropyl group, a 2-hydroxybutyl group, a 2-oxopropyl group, a 2-oxobutyl group, a 3-oxobutyl group, or a 2-oxopentyl group.

4. A therapeutic agent for a wound according to claim 1, wherein the compound represented by the general formula (I) is one compound selected from the group consisting of
({[5-hydroxy-2-({1-[4'-(hydroxymethyl)biphenyl-4-yl]piperidin-4-yl}methyl)-6-methylpyrimidin-4-yl]carbonyl}amino)acetic acid,
({[5-hydroxy-2-({1-[4'-(2-hydroxypropyl)biphenyl-4-yl]piperidin-4-yl}methyl)-6-methylpyrimidin-4-yl]carbonyl}amino)acetic acid,
[({5-hydroxy-2-[(1-{4'-[(2S)-2-hydroxypropyl]biphenyl-4-yl}piperidin-4-yl)methyl]-6-methylpyrimidin-4-yl}carbonyl)amino]acetic acid,
[({5-hydroxy-2-[(1-{4'-[(2R)-2-hydroxypropyl]biphenyl-4-yl}piperidin-4-yl)methyl]-6-methylpyrimidin-4-yl}carbonyl)amino]acetic acid,
({[5-hydroxy-6-methyl-2-({1-[4'-(2-oxopropyl)biphenyl-4-yl]piperidin-4-yl}methyl)pyrimidin-4-yl]carbonyl}amino)acetic acid, and
4'-[4-({4-[(carboxymethyl)carbamoyl]-5-hydroxy-6-methylpyrimidin-2-yl}methyl)piperidin-1-yl]biphenyl-4-carboxylic acid.

5. A therapeutic agent for a wound according to any one of claims 1 to 4, wherein the wound is selected from the group consisting of a skin ulcer, a leg ulcer, a burn ulcer, a frostbite ulcer, a traumatic ulcer, a pressure ulcer, a venous ulcer, an arterial ulcer, an immune ulcer, a postherpetic ulcer, a radiation induced ulcer, a drug ulcer, a diabetic ulcer, and a postoperative ulcer.

6. A therapeutic agent for a wound according to any one of claims 1 to 4, wherein the wound is selected from the group consisting of a burn ulcer, a pressure ulcer, and a diabetic foot ulcer.

7. A method for treating a wound, comprising administering a therapeutic agent for a wound according to any one of claims 1 to 6.

8. A method according to claim 7, wherein the wound is selected from the group consisting of a skin ulcer, a leg ulcer, a burn ulcer, a frostbite ulcer, a traumatic ulcer, a pressure ulcer, a venous ulcer, an arterial ulcer, an immune ulcer, a postherpetic ulcer, a radiation induced ulcer, a drug ulcer, a diabetic ulcer, and a postoperative ulcer.

9. A method according to claim 7, wherein the wound is selected from the group consisting of a burn ulcer, a pressure ulcer, and a diabetic foot ulcer.

10. A therapeutic agent for a wound according to any one of claims 1 to 6, wherein the therapeutic agent shortens a period required for the healing of the wound.

11. A therapeutic agent for a wound according to any one of claims 1 to 6 for use in a method for treating a wound.

12. A method for accelerating the healing of a wound, comprising administering a therapeutic agent for a wound according to any one of claims 1 to 6.
